Europäisches Patentamt

European Patent Office

Office européen des brevets

(11) Publication number: **0 147 224**
**A2**

## (12) EUROPEAN PATENT APPLICATION

(21) Application number: **84309066.3**

(22) Date of filing: **21.12.84**

(51) Int. Cl.⁴: **A 61 F 15/00**

(30) Priority: **22.12.83 GB 8334300**

(43) Date of publication of application:
**03.07.85 Bulletin 85/27**

(84) Designated Contracting States:
**AT BE CH DE FR IT LI LU NL SE**

(71) Applicant: **Burd, David Andrew Ross**
**124, Hinckley Road**
**Leicester Forest East Leicester, LE3 3JT(GB)**

(72) Inventor: **Burd, David Andrew Ross**
**124, Hinckley Road**
**Leicester Forest East Leicester, LE3 3JT(GB)**

(74) Representative: **Hallam, Arnold Vincent et al,**
**E.N. LEWIS & TAYLOR 144 New Walk**
**Leicester LE1 7JA(GB)**

(54) Surgical dressing aid.

(57) A device (10) for retaining a surgical dressing (22) in position over a skin defect which has been repaired using surgical threads (24) has an annular resilient ring (14) and a disc (12) which receive and grip the free ends of the threads (24) there between, the ring being seated in an annular recess (20) in the radially outer surface of the disc. The device is thus tensioned to the threads against the dressing to retain the dressing in position against the defect. This allows easy removal and replacement of the dressing for inspection of the skin defect.

Fig.1.

EP 0 147 224 A2

Title:   Surgical Dressing Aid

The present invention relates to surgical dressing aids.

The so-called 'tie over' dressing is commonly used in, for example, plastic surgery to maintain skin grafts in position.  Unfortunately, the 'tie over' dressing suffers from a number of disadvantages.  Normally, two people are required to apply a well-tensioned dressing and a certain amount of skill is required to tie such a dressing effectively.  Once the dressing is tied in place it is neither possible to adjust nor remove and replace the dressing for early inspection of the graft.  It is customary to leave the "tie-over" dressing undisturbed for a minimum of four days by which time the viability of the skin graft may have been irreversibly prejudiced by serous fluid or blood collecting between the skin graft and its bed.

The present invention seeks to provide an improved form of surgical dressing aid.

Accordingly, the present invention provides an  aid for retaining a surgical dressing in position over a skin defect which has been repaired using surgical threads, the aid comprising first and second means arranged to receive free ends of the threads therebetween and adapted to engage frictionally to grip said threads and inhibit removal thereof whereby said threads serve to tension the aid against the dressing and urge the dressing against the defect.

In a preferred embodiment of the present invention the first means is resilient and engages over the second means, the dimensions of the first and second means being such that the first means is maintained in tension when engaged over said second means.

Advantageously, the second means is a generally planer member while said first means is annular and engages in a recess in the radially outer surface of the second member, the threads being gripped between the two members.

Preferably the first member is a ring of resilient material while the second member is a disc with a recess of generally U-shaped cross section in its radially outer surface. Advantageously, one limb of the 'U' extends radially outwardly further than the other.

The present invention is further described hereinafter, by way of example, with reference to the accompying drawings, in which:

Fig. 1. is a cross section through a preferred embodiment of a surgical aid according to the present invention;

Fig. 2 - 6 show various stages in the application of the aid of Fig. 1 to a skin graft.

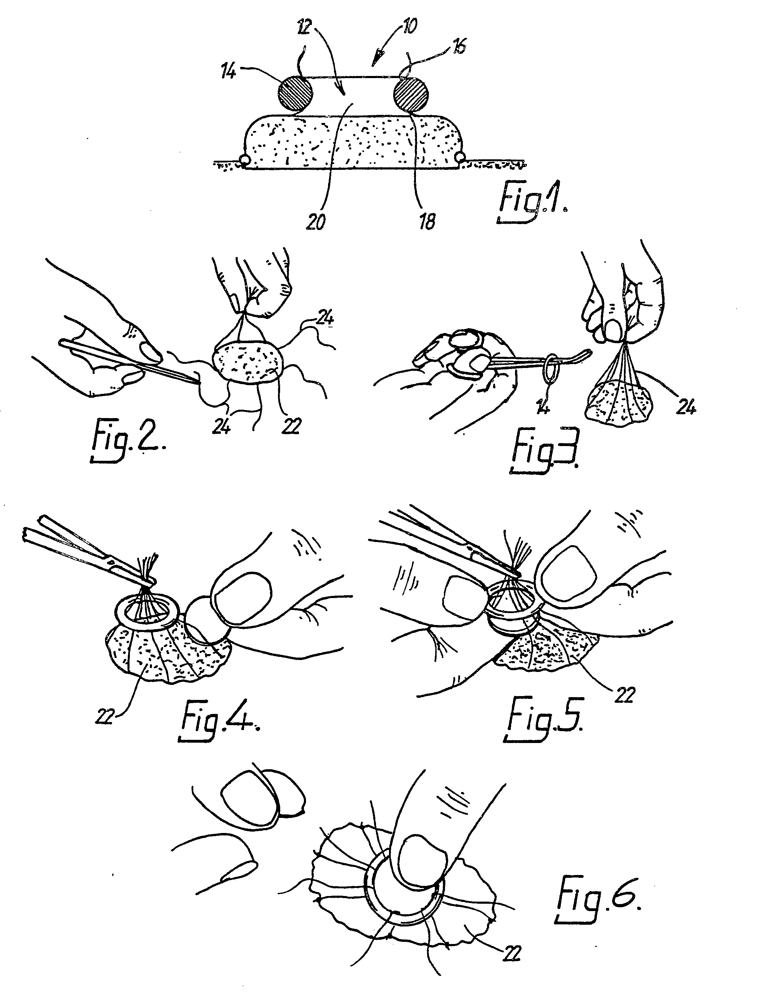

Fig.1 shows a dressing aid 10 in application, which comprises a flanged disc 12 and a ring 14 made of resilient material. The disc has upper and lower surfaces which radially extend into flanges 16 and 18 the result of which is to provide a channel or groove 20 in the radially outer surface of the disc, the groove 20 extending around the whole of the circumference of the disc. The groove 20 and the ring 14 are dimensioned such that the latter sits comfortably in the groove 20 with the inner diameter of the ring 14 being slightly less than the smallest diameter of the disc 12 to ensure that when the ring is in position in the groove 20 it is in a tensioned state.

As seen in Fig. 1, the lower flange 18 of the disc has a greater diameter than the upper flange 16. This is to allow the ring 14 to be slipped easily over the flange 16 into the groove 20 but to reduce the possibility of the ring also sliding easily over the flange 18.

Figs. 2 - 6 show the application of the dressing aid over a dressing 22 on a skin graft. The skin graft itself is applied and stitched in place with silk in the same way as for the conventional 'tie-over' dressing. To facilitate application of the dressing aid the silk threads are conveniently left longer than usual by, for example, 2cm. The skin graft is then dressed in the normal manner with a layer of paraffin gauze and proflavine wool padding. The free ends of the stitch threads 24 are then gathered together and held between the index finger and thumb of one hand (Fig.3) to allow the sealing ring 14 to be slipped over the threads 24. The flanged disc 12 is then introduced between the threads (Fig.4) with the smaller flange 16 uppermost and is then pressed into the ring 14 (Fig.5), thus clamping the threads between the ring and the disc (Fig. 6). The individual threads can then be pulled taut and arranged around the disc as shown in Fig. 6.

If the intention is that the graft is to be examined after a relatively short period of, for example, 48 hours then the threads can be left as they are, but if the dressing is to remain undisturbed for 5 days or more then the threads can be trimmed.

The disc can be made of any suitable material and can conveniently be injection moulded in plastics material. The ring is conveniently made of a resilient plastics material such as neoprene. Both the disc and the ring can be sterilised in the normal way.

Referring to Figure 3, the threads can be gathered and held together by, for example, an artery clip over which the ring 14 has already been slipped. This would leave both hands free at this stage.

The surgical aid illustrated in the drawings has the advantage of being easily and quickly applied, allowing for adjustment of the tension in the threads after application and can be removed and replaced easily and quickly at any time if inspection of the graft is desired. In addition, while it has been described with reference to skin grafts it will be appreciated that it can be used for retaining a surgical dressing in position over any time of skin defect, by use of surgical threads. It will also be appreciated that the disc and ring need not be circular but can be of any suitable shape, such as eliptical.

In addition, the disc itself need not be solid but can be hollow, e.g. in the form of a ring and can be resilient.

CLAIMS:

1. A device for retaining a surgical dressing (22) in position over a skin defect which has been repaired using surgical threads (24) characterised in that the device comprises first (14) and second (12) means arranged to receive free ends of the threads (24) therebetween and adapted frictionally to engage and grip said threads (24) whereby said threads (24) serve to tension the device (10) against the dressing (22) and retain the dressing in position against the defect.

2. A device according to claim 1 wherein said first means (14) is resilient and engages over said second means (12), the dimensions of said first and second means being such that the first means (14) is maintained in tension when engaged over said second means (12).

3. A device as claimed in claim 1 wherein said second means (12) is resilient and engages within said first means (14), the dimensions of said first and second means being such that the second means (12) is maintained in compression when engaged in said first means (14).

4. A device as claimed in claims 1 to 3 wherein said second means (12) is a generally planar member while said first means (14) is annular and engages in a seating means (20) formed in the radially outer surface of the second member (12), the threads (24) being gripped between the two members.

5. A device as claimed in claim 4 wherein said seating means (20) is an annular recess.

6. A device as claimed in any of claims 1 to 5 wherein said first member (14) is a ring of resilient material while the second member (12) is a substantially planar member (20) of generally U-shaped cross section in its radially outer surface.

7. A device as claimed in claim 6 wherein one limb (18) of the U-shaped recess (20) extends radially outwardly further than the other.

Fig.1.

Fig.2.

Fig3.

Fig.4.

Fig.5.

Fig.6.